# EUROPEAN PATENT APPLICATION

(11) **EP 2 491 903 A1**
(43) Date of publication of application: **29.08.2012**
(21) Application number: 10825047.3
(22) Date of filing: 22.10.2010
(51) Int. Cl.: A61F 9/007

(54) **TUBE DEVICE FOR INSERTION INTO LACRIMAL PASSAGE**

(30) Priority: 22.10.2009 JP 2009243922
(71) Applicant: Kaneka Corporation, Osaka-shi, Osaka 530-8288 (JP)
(72) Inventor: FUKAYA, Kohei, Settsu-shi Osaka 566-0072 (JP); NAKANO, Ryoji, Settsu-shi Osaka 566-0072 (JP); MIYAUCHI, Hidekazu, Settsu-shi Osaka 566-0072 (JP)
(74) Representative: TBK
(86) International application number: PCT/JP2010/068689
(87) International publication number: WO 2011/049198

(57) **Abstract**

A tube device for insertion into a lacrimal passage, optimized according to the structure of a lacrimal passage and capable of being operated without impairing the functional effect of a lacrimal passage endoscope. A tube device for insertion into a lacrimal passage comprises a pair of tubes to be placed in the lacrimal passage. Openings are formed at both ends of the pair of tubes. A reinforcement body is disposed and held in the vicinity of each opening at a position located at a predetermined distance from the opening.

## Description

### Technical Field

The present invention relates to a tube device for insertion into a lacrimal passage used for treatment of lacrimal passage obstruction.

### Background Art

Examples of the treatment of lacrimal passage obstruction that causes epiphora include (i) probing by a lacrimal passage bougie, (ii) placement of a tube device for insertion into the lacrimal passage, (iii) dacryocystorhinostomy (DCR), (iv) lacrimal canaliculoplasty, (v) nasolacrimal duct plasty, and (vi) lacrimal caruncle moving surgery.

The probing by a lacrimal passage bougie (i) opens an obstruction area by insertion of a narrow tube called a bougie into a lacrimal passage and reconstructs a flow path of tears. This treatment is performed as the first treatment because it is easy and minimally invasive. The dacryocystorhinostomy (DCR) (iii), the lacrimal canaliculoplasty (iv), the nasolacrimal duct plasty (v), and the lacrimal caruncle moving surgery (vi) are highly effective but relatively highly invasive treatments because such a treatment makes an incision in the face or makes a hole in the bone. Hence, such a treatment is performed as the last means.

The tube device for insertion into the lacrimal passage (such as a lacrimal passage tube) used for the placement of a tube device for insertion into the lacrimal passage (ii) is placed in order to maintain the flow path and to reconstruct the tissue after the probing by a lacrimal passage bougie (i). The placement of a tube device for insertion into the lacrimal passage (ii) is easier and less invasive and has larger effect comparing with each treatment method of (iii) to (vi) and thus is widely performed around the world. Specifically, so-called a nunchaku-type lacrimal passage tube (for example, see Fig. 1) as described in Patent Document 1 has been widely used, and in the nunchaku-type lacrimal passage tube, the center part of the tube is formed from a thin and soft tube or rod and each end is formed from a hard and thick tube.

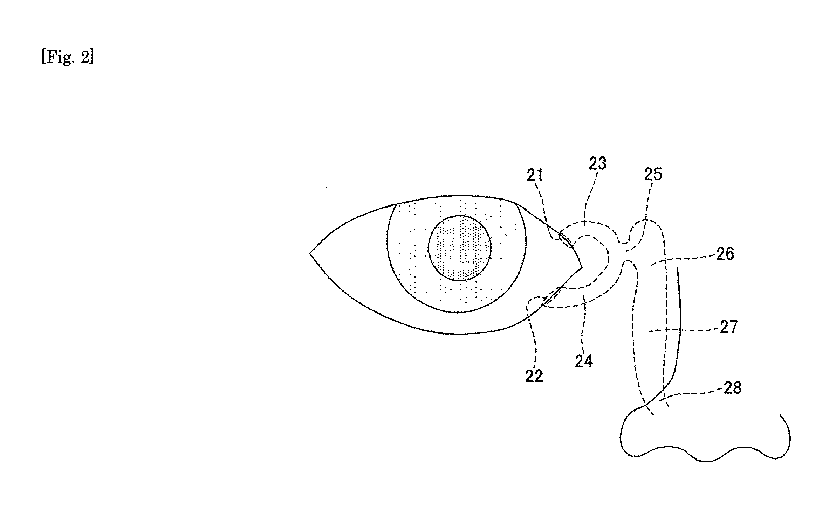

The nunchaku-type lacrimal passage tube includes a tube and a pair of bougies, and the bougie is inserted from a slit on each end of the tube. The bougie is operated to introduce the tube into the lacrimal passage for placement. As shown in Fig. 2, the lacrimal passage includes the lacrimal puncta (21, 22), the lacrimal canaliculi (23, 24), the lacrimal sac (26), the nasolacrimal duct (27), and the like. Into the lacrimal passage, the nunchaku-type lacrimal passage tube is inserted.

However, in order to insert the nunchaku-type lacrimal passage tube, the operation is fumbled in the lacrimal passage and the bougie is operated blindly. Hence, such an operation may break the tube or may make a hole on an area other than the normal lacrimal passage (a false passage) to give poor treatment results.

On this account, in recent years in order to avoid such a trouble, the operation is often carried out using a lacrimal endoscope for the observation of the lacrimal passage.
However, even when the lacrimal endoscope is used for the insertion of the lacrimal passage tube, the safety is not sufficiently secured. This is because the lacrimal endoscope must be removed out from the body in order to insert the lacrimal passage tube after the observation in the lacrimal passage using the lacrimal endoscope, the operation cannot be performed while observing a correct position and the like on a monitor of the lacrimal endoscope, and, as a result, the bougie is blindly operated to give insufficient effect.
Furthermore, the tube has blind ends, and hence a flow examination cannot be performed through the tube.

### Citation List

### Patent Literature

Patent Document 1: Japanese Patent No. 2539325

### Summary of Invention

### Technical Problem

In view of the above problems, an object of the present invention is to provide a tube device for insertion into a lacrimal passage that is optimized based on the structure of the lacrimal passage and to provide a tube device for insertion into a lacrimal passage that can be operated without impairing the functional effect of a lacrimal endoscope.

### Solution to Problem

The inventors have intensively studied in order to solve the problems. As a result, the inventors have found that a tube device for insertion into a lacrimal passage, the tube device having an opening at each end of an integrated tube as the component of the tube device and having a reinforcement body near the opening at a position located at a predetermined distance from the opening, can use a lacrimal endoscope in combination, can secure a visual field of the lacrimal endoscope, and can remove the lacrimal endoscope after the use and place the tube device for insertion into a lacrimal passage (such as a lacrimal passage tube) at a position where a health professional desires, and the invention has been accomplished.

Namely, the summary of the present invention is as below.

[1] A tube device for insertion into a lacrimal passage includes an integrated tube to be placed in the lacrimal passage. The integrated tube includes two ends, each end being formed with an opening, a reinforcement body is disposed and held near the opening, and a disposed position of the reinforcement body is located at a predetermined distance from the opening.

[2] The tube device for insertion into a lacrimal passage according to the item [1] further includes a lock means for disposing and holding the reinforcement body.

[3] In the tube device for insertion into a lacrimal passage according to the item [1] or [2], each end of the integrated tube has an edgeless shape.

[4] In the tube device for insertion into a lacrimal passage according to any one of the items [1] to [3], a constituent material of the reinforcement body is stainless steel.

[5] In the tube device for insertion into a lacrimal passage according to any one of the items [1] to [4], a constituent material of the integrated tube includes a resin composition of an isobutylene block copolymer (A) and a thermoplastic polyurethane resin (B) at a weight ratio of (A)/(B) = 1/99 to 99/1.

[6] In the tube device for insertion into a lacrimal passage according to any one of the items [1] to [5], the opening has a diameter of 0.5 to 0.8 mm.

[7] In the tube device for insertion into a lacrimal passage according to any one of the items [1] to [6], the predetermined distance is 2 mm or less.

### Advantageous Effects of Invention

The tube device for insertion into a lacrimal passage of the present invention has the openings in the tube and hence can secure the visual field of a lacrimal endoscope through the opening and can perform a flow examination. The visual field can be secured from a lacrimal endoscope. Thus, a pathway in which the tube passes can be surely identified to avoid troubles, for example, the tube makes a false passage and damages a mucosa and the like to cause bleeding. The tube can be smoothly inserted into the lacrimal passage using the force to a lacrimal endoscope. The reinforcement body works as a stopper to reduce the risk of pushing a lacrimal endoscope out from the opening. In addition, the tube device includes an integrated tube, and thus the tube can be inserted into both of the upper and lower lacrimal passages that are a specific structure to the lacrimal passage.

### Brief Description of Drawings

Fig. 1 is a schematic view showing a conventional nunchaku-type lacrimal passage tube.
Fig. 2 is an explanatory view showing an anatomical structure of the lacrimal passage.
Fig. 3(a) is a cross-sectional view showing an example of a placement part of the tube device for insertion into a lacrimal passage of the present invention.
Fig. 3(b) is a cross-sectional view showing a leading end in the example in Fig. 3(a) with a lacrimal endoscope inserted.
Fig. 3(c) is a cross-sectional view showing another example of the leading end of the placement part of the tube device for insertion into a lacrimal passage of the present invention.
Fig. 4 is a schematic view for explaining a measurement method of the push out strength of the reinforcement body and the tube in examples.

### Description of Embodiments

The present invention will be described in detail hereinafter.

The lacrimal passage in the present invention includes, as shown in Fig. 2, the superior/inferior lacrimal puncta (21/22), the superior/inferior lacrimal canaliculi (23/24), the common canaliculus (25), the lacrimal sac (26), the nasolacrimal duct (27), the nasal cavity (not shown in the drawing), and Hasner's valve (not shown in the drawing), and is a duct (an accessory organ of the eye) that delivers a tear fluid produced from the lacrimal gland (not shown in the drawing) from the eye surface to the inferior nasal meatus (28). Fig. 2 is a schematic anatomical structure of the lacrimal passage. The duct from the superior lacrimal punctum (21) through the superior lacrimal canaliculus (23) and the common canaliculus (25) to the inferior nasal meatus (28) is called the superior lacrimal passage, while the duct from the inferior lacrimal punctum (22) through the inferior lacrimal canaliculus (24) and the common canaliculus (25) to the inferior nasal meatus (28) is called the inferior lacrimal passage.

The tube device for insertion into a lacrimal passage of the present invention includes an integrated tube to be placed in the lacrimal passage.
The term "integrated" means that two tubes to be correspondingly inserted into the superior/inferior lacrimal passages are connected to be one tube as a whole.
The integrated tube means a soft tube device for insertion that is inserted for the reconstruction treatment of the lacrimal passage and that has a predetermined length.

The integrated tube has both ends each formed with an opening. The tube has the openings in this manner, and thus through the opening, the visual field can be secured from a lacrimal endoscope and a flow examination can be performed.

Examples of the structure of the integrated tube include a cylindrical tube having both ends formed with openings and having a hollow portion through which the openings communicate with each other. Examples of the cylindrical tube include a tube having substantially constant inner and outer diameters over the full-length and a tube including a center part having a substantially constant small diameter.

Other examples of the structure of the integrated tube include a tube including a center part in a rod shape and, at each end, a cylindrical part having an end with an opening and having a hollow portion that continues to the opening. In this case, the integrated tube may have a substantially constant outer diameter over the full-length or may have a substantially constant small diameter part in the center part in a rod shape.

Among the integrated tubes exemplified above, more specifically, the tube including the center part having a substantially constant small diameter part may employ the cylindrical part at each end as the part for placing from the lacrimal sac to the nasolacrimal duct and may employ the part in a rod shape for placing from the lacrimal punctum to the lacrimal canaliculus. The tube device for insertion into a lacrimal passage having such a shape and such a structure and having the center part that is softer than both side parts is referred to as a so-called nunchaku-type lacrimal passage tube as described above.

The nunchaku-type lacrimal passage tube means a lacrimal passage tube that is named after a nunchaku used in martial arts in China because the shape is similar to that of the nunchaku. A preferred tube has such a soft center part as the tube becomes a reversed U-shape when the tube is held up while holding the center point in a longitudinal direction of the tube.

The shape of each end of the integrated tube is not specifically limited but is preferably an edgeless shape in order to prevent damage to the lacrimal passage. Examples of the shape of each end include, but are not necessarily limited to, an obtuse outer shape such as substantially a cone shape and a pyramid shape, a chamfered edge shape, and a radius shape.

In the present invention, the integrated tube may include an insert part for inserting a bougie or a lacrimal endoscope. In particular, in many cases of the so-called nunchaku-type lacrimal passage tube, commonly, a bougie or a lacrimal endoscope cannot be inserted into the center part. Hence, in order to insert a lacrimal endoscope or the like into the hollow portion of the cylindrical part that is provided on each side of the center part, an insert part is required to be provided for the continuity between the outside and the hollow portion. The insert part is preferably provided on a side wall of the cylindrical part. The structure of the insert part is not specifically limited and may be properly selected from a small hole, a slit, and the like.

The constituent material (material) of the integrated tube is not specifically limited, and examples include, but are not necessarily limited to, resin compositions containing silicone, polyurethane, an isobutylene copolymer, an alloy of them, and the like.
In the present invention, the alloy is not specifically limited, but when an alloy of polyurethane and an isobutylene copolymer is used, the control of the ratio of the isobutylene block copolymer (A) and the thermoplastic polyurethane resin (B) can achieve the hardness control of the tube. The thermoplastic polyurethane resin (B) having a larger ratio can increase the hardness of the tube. From the viewpoint of anti-thrombogenic properties, surface slidability, and flexibility, the isobutylene block copolymer (A) is preferably contained in an amount of 1% by weight or more (namely, the ratio (A)/(B) of the isobutylene block copolymer (A) and the thermoplastic polyurethane resin (B) is preferably 1/99 to 99/1 in a weight ratio). Specifically, from the viewpoint of abrasion resistance, the ratio (A)/(B) of the isobutylene block copolymer (A) and the thermoplastic polyurethane resin (B) is preferably 1/99 to 70/30 in a weight ratio. In particular, from the viewpoint of compressive stress, the ratio (A)/(B) of the isobutylene block copolymer (A) and the thermoplastic polyurethane resin (B) is preferably 1/99 to 50/50 in a weight ratio. The resin composition for the integrated tube used in the present invention may be composed of the isobutylene block copolymer (A) and the thermoplastic polyurethane resin (B) alone and may be mixed with other components.

The isobutylene block copolymer (A) is preferably "SIBSTAR102T" that is a styrene-isobutylene-styrene block copolymer (hereinafter, also referred to as SIBS) and is manufactured by Kaneka Corporation. Preferred examples of the thermoplastic polyurethane resin (B) (hereinafter, also referred to as TPU) include "Miractran E385PNAT" manufactured by Nippon Miractran Co, Ltd. and "Tecothane TT1074A" manufactured by Noveon that are ether aromatic ring polyurethanes, "Tecoflex EG100A" and "Tecoflex EG85A" that are manufactured by Noveon and are ether alicyclic polyurethanes, and "Carbothane PC3575A" that is manufactured by Noveon and is a polycarbonate polyurethane.

In the present invention, a reinforcement body is disposed and held near the opening formed at each end of the integrated tube. The reinforcement body may be disposed near at least one of the openings formed on both ends of the integrated tube. Namely, the reinforcement body may be disposed near one opening of the integrated tube and may be disposed near the openings at both ends. For example, for the case that the tube device for insertion into a lacrimal passage is placed through one of the superior/inferior lacrimal canaliculi and for the case that the tube device for insertion into a lacrimal passage is inserted from the common canaliculus to be placed due to a scarring of the superior/inferior lacrimal canaliculi, the reinforcement body may be disposed near one of the openings formed at both ends of the integrated tube, while for the case that one tube device for insertion into a lacrimal passage is placed through both the superior/inferior lacrimal canaliculi, the reinforcement bodies may be disposed near the openings formed at both ends of the integrated tube. In the former case, the insert part for inserting an endoscope and the like may be formed so that such an apparatus is inserted from the opening without disposing the reinforcement body.
For the case that the reinforcement body is disposed near the opening formed at each end of the integrated tube, in combination of, for example, a disposed position described later of the reinforcement body, the tube can be easily placed in the superior/inferior lacrimal passages using one tube device for insertion into a lacrimal passage.

The shape of the reinforcement body is not specifically limited as long as the reinforcement body can be disposed near the opening of the integrated tube, can secure a visual field of the lacrimal endoscope inserted in the hollow portion of the tube, and can work as the stopper for the lacrimal endoscope. Examples of the shape may include a substantially ring shape.

The disposed position of the reinforcement body is set so as to be near the opening and to have a predetermined distance from the opening (the most end position of the opening). The predetermined distance is determined from the viewpoints of the role as the stopper for a lacrimal endoscope and securement of the visual field of the lacrimal endoscope. For example, in order to secure the visual field of the lacrimal endoscope, the position of a lens at the leading end of the lacrimal endoscope is preferably 2 mm or less from the most end position of the tube opening. In order to secure a visual field range of the lacrimal endoscope of 70% or more, the position of a lens at the leading end of a lacrimal endoscope is more preferably 1.5 mm or less and even more preferably 1 mm. Therefore, from the viewpoint of the securement of the visual field of the lacrimal endoscope, the predetermined distance is preferably 2 mm or less, more preferably 1.5 mm or less, and even more preferably 1 mm or less, from the opening (the most end position of the opening).

The visual field range of a lacrimal endoscope can be also affected by the diameter of the opening (especially, the diameter of the end of the opening) except for a so-called viewing angle of the lacrimal endoscope. The diameter of the opening (also referred to the opening diameter) is preferably as large as possible from the viewpoint of the securement of the visual field of the lacrimal endoscope. Meanwhile, the opening having a larger diameter reduces the wall thickness of the end part of the tube and thus the reinforcement body is difficult to be held. Hence, when the tube is inserted into the lacrimal passage using the force to a lacrimal endoscope, the reinforcement body may be pushed out from the opening. To address this, in order to hold the reinforcement body to prevent the pushing out and to secure the visual field of the lacrimal endoscope, the diameter of the opening (opening diameter) is preferably 0.5 to 0.8 mm and more preferably 0.65 to 0.75 mm.

As mentioned above, the reinforcement body is disposed near the opening at the end of the tube at a position located at a predetermined distance from the opening, and hence the tube can be smoothly inserted into the lacrimal passage using the force to a lacrimal endoscope while securing the visual field of the lacrimal endoscope. The accidental risk of pushing a lacrimal endoscope out from the opening can be also reduced because the reinforcement body works as a stopper. Such an effect can be further increased by the control of the opening diameter of the opening to the above range in addition to the distance from the opening.
During a lacrimal endoscope is used, a pathway in which the tube passes can be surely ascertained to reduce troubles, for example, the tube makes a false passage and damages a mucosa and the like to cause bleeding.
The reinforcement body reinforces a vicinity of the tube end, and thus the opening processing may be performed as an after-processing.

The constituent material of the reinforcement body is not specifically limited, and examples include various hard resins and metals such as stainless steel. Stainless steel is preferred in order to prevent corrosion due to the contact with a body fluid, a drug solution, and the like.

In the present invention, a lock means for disposing and holding the reinforcement body may be formed. Such a lock means can hold the reinforcement body more reliably near the opening of the tube and the reinforcement body can reliably work as the stopper for a lacrimal endoscope. The lock means is preferably formed on an inner wall constituting the cylindrical hollow portion of the integrated tube. The structure of the lock means is not specifically limited as long as it can reliably hold the reinforcement body, and examples include a groove and a protrusion that are formed so as to lock the reinforcement body in the cylindrical inner wall.

In order to increase insertion properties into the lacrimal passage, of the tube device for insertion into a lacrimal passage, the tube may have an outer side with a hydrophilic coating. The coating provides lubricating properties when the tube is in contact with blood and reduces the resistance when the tube is inserted. The hydrophilic coating is not specifically limited and preferably used examples include hydrophilic polymers such as poly(2-hydroxyethyl methacrylate), polyacrylamide, polyvinylpyrrolidone, and polyethylene glycol and a blend of them.

Hereinafter, the tube device for insertion into a lacrimal passage of the present invention will be described based on an embodiment shown in drawings, but the present invention is not intended to be limited to the embodiment.

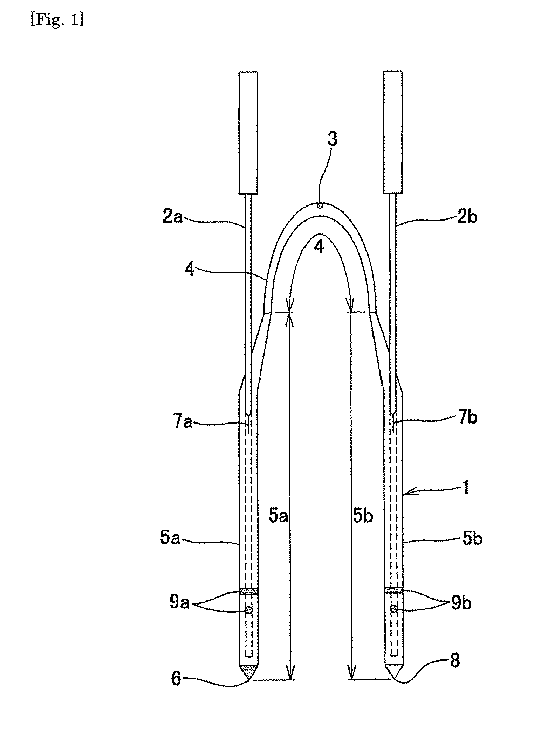

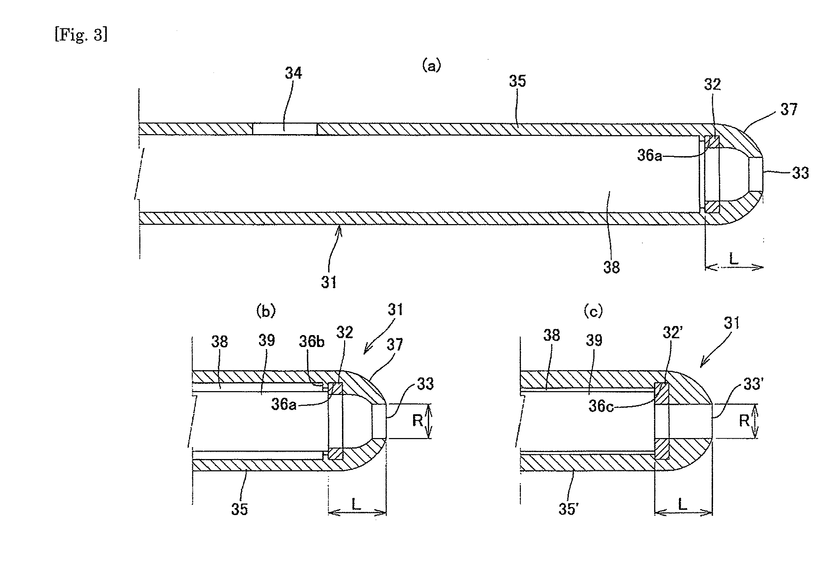

Fig. 1 shows an example of a conventional nunchaku-type lacrimal passage tube (with bougies inserted), and Fig. 3(a) is a schematic cross-sectional view of an embodiment of a tube part to be placed in the inferior nasal meatus and the like of the lacrimal passage, in the tube device for insertion into a lacrimal passage of the present invention.
Fig. 3(b) is a schematic cross-sectional view of the end of the tube part with a lacrimal endoscope inserted in the hollow portion of the tube part in the example shown in Fig. 3(a).
Fig. 3(c) is a schematic cross-sectional view showing another example of the end of the tube part. In Fig. 3(c), a lacrimal endoscope is inserted in the hollow portion of the tube part as with Fig. 3(b).

First, the conventional nunchaku-type lacrimal passage tube shown in Fig. 1 will be described.
The conventional nunchaku-type lacrimal passage tube 1 includes a cylinder-shaped or rod-shaped center part 4 that is disposed at an approximately center part and that is to be placed in the lacrimal punctum, the lacrimal canaliculus, the common canaliculus, and the lacrimal sac, a cylinder-shaped first cylinder part 5a that is formed continuously at one end of the center part 4 and that is to be placed in the lacrimal sac, the nasolacrimal duct, the Hasner's valve, and the inferior nasal meatus, and a cylinder-shaped second cylinder part 5b that is formed continuously at the other end of the center part 4 and that is to be placed in the lacrimal sac, the nasolacrimal duct, the Hasner's valve, and the inferior nasal meatus. The center part 4 is formed to have a smaller diameter than those of the first cylinder part 5a and the second cylinder part 5b, and hence the lacrimal passage tube has a so-called nunchaku shape.

The first cylinder part 5a has a first end 6, while the second cylinder part 5b has a second end 8, and each end is blind and has an acute leading end. The leading end part containing the first end 6 is colored in order that an operator can easily distinguish it from the leading end part containing the second end 8. The first cylinder part 5a has two marks 9a at positions located at predetermined distances from the first end 6, so that an operator can visually identify an insertion depth. The second cylinder part 5b has marks 9b similarly.

The first cylinder part 5a and the second cylinder part 5b have slits 7a and 7b for inserting bougies, on side walls near the center part 4, respectively. Through the slits 7a and 7b, a first bougie 2a and a second bougie 2b are inserted into the hollow portions in the first cylinder part 5a and the second cylinder part 5b, respectively.

The center part 4 has a midpoint 3 at a substantially center position of the nunchaku-type lacrimal passage tube for easily ascertaining of a insert position and a placement position.

In the case that the tube device for insertion into a lacrimal passage of the present invention is a nunchaku-type lacrimal passage tube, the basic structure is the same as that in Fig. 1. There are differences in the structures of the first cylinder part 5a and the second cylinder part 5b. These differences will be described below. The tube device for insertion into a lacrimal passage of the present invention can employ other components than the differences, such as the mark 9a (9b) and the coloring of the end in Fig. 1.

The nunchaku-type lacrimal passage tube that is an embodiment of the tube device for insertion into a lacrimal passage of the present invention employs a tube having a cylinder-shaped structure as shown in Fig. 3(a) in place of the first cylinder part 5a and the second cylinder part 5b in the nunchaku-type lacrimal passage tube 1 shown in Fig. 1. Namely, the tube shown in Fig. 3(a) is formed continuously to each end of the center part 4 shown in Fig. 1 in a common procedure to make the tube device for insertion into a lacrimal passage having an integrated tube of the present invention.
Fig. 3(a) shows a cross-sectional view of one tube having a cylinder-shaped structure of the integrated tube, but the other tube has substantially the same structure, which will not be described.

As shown in Fig. 3(a), in the nunchaku-type lacrimal passage tube 31 as an embodiment of the tube device for insertion into a lacrimal passage of the present invention, a cylinder part 35 has an end opening 33 at an end and disposes and holds a reinforcement body 32 near the end opening 33 at a position located at a predetermined distance L from the end opening 33. The predetermined distance L is a distance between the end face of the reinforcement body 32 opposite to the end opening 33 and the end opening 33. The reinforcement body 32 has a ring shape and is fit and fixed to a groove 36a and a protrusion 36b that are provided on an inner peripheral face of the inner wall of the cylinder part 35 as a lock means. The end of the cylinder part 35 has a radius part 37 to make an edgeless shape. The cylinder part 35 further has a slit 34 on a side wall at a position near the center part. Through the slit 34, the outside communicates with a hollow portion 38, and a bougie or a lacrimal endoscope can be inserted.

As shown in Fig. 3(b), a lacrimal endoscope 39 inserted into the hollow portion 38 in the cylinder part 35 through a slit (not shown in the drawing) is brought into contact with the end face of the reinforcement body 32 opposite to the end opening 33. Thus, the protrusion of the lacrimal endoscope 39 from the end opening 33 can be prevented and, using the force loaded to the lacrimal endoscope 39, the cylinder part 35 can be readily inserted into the lacrimal passage. A distance from the leading end face of the lacrimal endoscope 39 inserted into the hollow portion 38 to the end opening 33 is set as the predetermined distance L, and thus the visual field of the lacrimal endoscope 39 can be secured. Namely, the predetermined distance L is a distance that is required for securing the visual field of the lacrimal endoscope 39, and it is affected by an opening diameter R of the end opening 33 except for a so-called viewing angle of the lacrimal endoscope 39.
In the present example, the end part has an inner structure where the inner diameter of the hollow portion increases toward the direction from the end opening 33 to the center part 4 (the left direction in Fig. 3(b)) to correspond to the inner diameter of the ring-shaped reinforcement body 32.

Fig. 3(c) shows another structure example of the end part of the cylinder part 35 in the nunchaku-type lacrimal passage tube 31 as an embodiment of the tube device for insertion into a lacrimal passage of the present invention. In the present example, the end part has the same constitution as that in Fig. 3(b) except for the inner structure, the structure of the lock means, and the structure of the reinforcement body. In the inner structure in the end part of the present example, the hollow portion has a constant inner diameter that is the same as the opening diameter R toward the direction from the end opening 33' to the center part 4 (the left direction in Fig. 3(c)). Thus, the ring-shaped reinforcement body 32' has a smaller inner diameter than that of the reinforcement body 32 in Fig. 3(b). In the device having such a structure, the cylinder body from the reinforcement body 32' to the end has a larger wall thickness than that of the cylinder body from the reinforcement body 32' to the central part. Hence, such a device is likely to improve the prevention effect on pushing out from the reinforcement body 32' when a lacrimal endoscope is inserted for pushing, comparing to the case of the inner structure in Fig. 3(b).
The reinforcement body 32' is fit and fixed to a groove 36c (lock means) provided on an inner wall of the cylinder part 35'. The tube has no protrusion 36b as shown in Fig. 3(b).
As with in Fig. 3(b), a distance from the leading end face of a lacrimal endoscope 39 inserted into the hollow portion 38 to the end opening 33' is set as a predetermined distance L.

### Examples

### (Production Examples 1 to 10)

Predetermined amounts of a styrene-isobutylene block copolymer (hereinafter referred to as SIBS) and polyurethane (Tecoflex EG85A; manufactured by Noveon) were prepared at a weight ratio of 10:90, and the whole was kneaded with a twin screw extruder (φ 4.0, L/D = 40, a rotation speed of 500 rpm). The obtained SIBS/polyurethane kneaded product having a Shore hardness of 72A was extrusion-molded to produce a tube a (an inner diameter of 0.90 mm and an outer diameter of 1.3 mm) and a tube b (an inner diameter of 0.90 mm and an outer diameter of 1.4 mm).
Each of the produced tubes a and b was cut to have a predetermined length, and, using an SUS core material having an obtuse leading end, an SUS ring (an inner diameter of 0.8 mm, an outer diameter of 1.0 mm, a width and height of 0.5 mm, SUS304) as the reinforcement body was inserted into each hollow portion of the tubes a and b to be disposed at a position located at 1 to 1.5 mm from the end of each tube.
Through each hollow portion of the tubes a and b disposing the SUS ring, an SUS core material (for tube holding) having an outer diameter of 0.49 mm was inserted. Next, a vicinity of the area where the SUS ring was disposed in each of the tubes a and b was inserted through a shrink tube (manufactured by Raychem, RNF-100-3/64), and using a hot air adhesion apparatus set at 200°C, the shrink tube part was locally heated to melt each of the tubes a and b and to fix the SUS ring. Consequently, the SUS ring was fixed on the inner wall in each of the tubes a and b while maintaining the inner diameter of the end part of each of the tubes a and b to be about 0.9 mm.
Next, from each end of the tubes a and b that disposed and held the SUS ring as above, an SUS core material having a predetermined outer diameter was inserted so that the end opening would have a desired inner diameter R (0.5/0.6/0.7/0.75/0.8 mm).
Next, in a local heater (manufactured by Nippon Avionics Co., Ltd., TCW-215), an edge part at each end part of the tubes a and b through which a desired SUS core material was inserted was set in a mold having a radius shape. The end part was heated at 100°C for 50 seconds and then at 95°C for 5 seconds to process the leading end into the radius shape. As a result, tubes that had tube inner and outer diameters and end part sizes (predetermined distance L and opening diameter R of the end opening) shown in Table 1 and that constituted the tube device for insertion into a lacrimal passage of the present invention were produced. The cross sectional structure of the end part was substantially the structure as shown in Fig. 3(c) and had the predetermined distance L, the opening diameter R, the groove as the lock means, the radius-shaped end, and the like.

### (Evaluation)

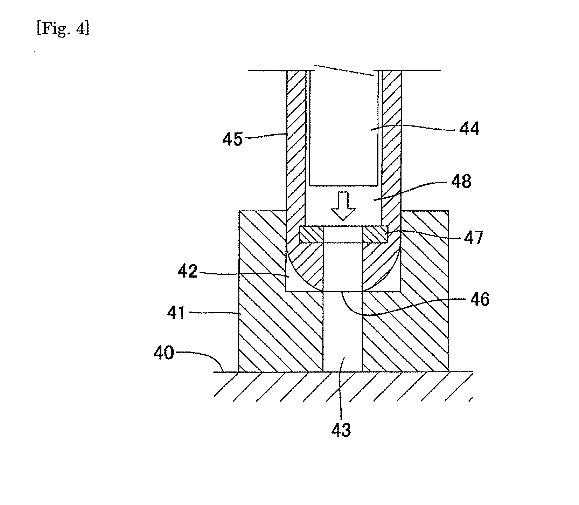

Using each tube of Production Examples 1 to 10 constituting the tube device for insertion into a lacrimal passage of the present invention, a lacrimal endoscope was insert into the hollow portion in the tube to evaluate the pushing out strength from the tube opening, of the SUS ring as the reinforcement body when the tube device for insertion into a lacrimal passage of the present invention was inserted into the lacrimal passage. The evaluation method is as follows.
As shown in Fig. 4, on a horizontal table 40, a cylinder-shaped push-out jig 41 equipped with coaxial hollow portions (42, 43) having larger and smaller inner diameters was placed, and the end part of each tube of Production Examples 1 to 10 was fit into the hollow portion 42 having a larger inner diameter so that the end of each tube pointed downward in Fig. 4. The hollow portion 42 has such an inner diameter as the tube 45 can slide on the basis of the outer diameter of the tube 45, while the smaller hollow portion 43 has an inner diameter that is substantially the same as the opening diameter R of the end opening 46. A transitional part from the hollow portion 42 to the hollow portion 43 includes a face parallel to a horizontal surface of the horizontal table 40.
Next, into the hollow portion 48 of the tube 45, a pusher 44 (a model member for a lacrimal endoscope) having an outer diameter of 0.9 mm was inserted, and using a compression tester (manufactured by Shimadzu Corporation, EZ-TEST), the maximum resistance load was determined when the pusher 44 was moved downward (toward the arrow direction) in Fig. 4. The measurement was carried out at a movement speed of the pusher 44 of 20 mm/min, an environmental temperature of 23°C, and an environment humidity of 50% RH. The measurement results are shown in Table 1. For the criteria, a tube having a maximum resistance load of 1.0 N or more was regarded as good.

**[Table 1]**

| | Tube outer diameter/ tube inner diameter [mm/mm] | Distance L [mm] | Opening diameter R of end opening [mm] | Maximum resistance load [N] |
|---|---|---|---|---|
| Production Example 1 | 1.4/0.9 | 1.5 | 0.50 | 6.6 |
| Production Example 2 | 1.4/0.9 | 1.5 | 0.60 | 5.0 |
| Production Example 3 | 1.4/0.9 | 1.5 | 0.70 | 5.9 |
| Production Example 4 | 1.4/0.9 | 1.5 | 0.75 | 4.8 |
| Production Example 5 | 1.4/0.9 | 1.5 | 0.80 | 5.0 |
| Production Example 6 | 1.3/0.9 | 1.5 | 0.50 | 4.9 |
| Production Example 7 | 1.3/0.9 | 1.5 | 0.60 | 4.6 |
| Production Example 8 | 1.3/0.9 | 1.5 | 0.70 | 4.7 |
| Production Example 9 | 1.3/0.9 | 1.5 | 0.75 | 3.0 |
| Production Example 10 | 1.3/0.9 | 1.0 | 0.70 | 4.8 |

- 1: Tube device for insertion into a lacrimal passage (nunchaku-type lacrimal passage tube)
- 2a: First bougie
- 2b: Second bougie
- 3: Midpoint
- 4: Center part
- 5a: First cylinder part
- 5b: Second cylinder part
- 6: First end
- 7: Slit for insertion of a bougie
- 8: Second end
- 21: Sperior lacrimal punctum
- 22: Inferior lacrimal punctum
- 23: Superior lacrimal canaliculus
- 24: Inferior lacrimal canaliculus
- 25: Common canaliculus
- 26: Lacrimal sac
- 27: Nasolacrimal duct
- 28: Inferior nasal meatus
- 31: Tube device for insertion into a lacrimal passage (nunchaku-type lacrimal passage tube)
- 32: Reinforcement body
- 33: End opening
- 34: Slit
- 40: Table
- 41: Push-out jig
- 42: Hollow portion having a larger inner diameter
- 43: Hollow portion having a smaller inner diameter
- L: Predetermined distance
- R: Opening diameter

## Claims

1. A tube device for insertion into a lacrimal passage, the tube device comprising an integrated tube to be placed in the lacrimal passage;
the integrated tube including two ends, the end being formed with an opening,
a reinforcement body being disposed and held near the opening, and
a disposed position of the reinforcement body being located at a predetermined distance from the opening.

2. The tube device for insertion into a lacrimal passage according to claim 1 further comprising a lock means for disposing and holding the reinforcement body.

3. The tube device for insertion into a lacrimal passage according to claim 1 or 2, wherein each end of the integrated tube has an edgeless shape.

4. The tube device for insertion into a lacrimal passage according to any one of claims 1 to 3, wherein a constituent material of the reinforcement body is stainless steel.

5. The tube device for insertion into a lacrimal passage according to any one of claims 1 to 4, wherein a constituent material of the integrated tube includes a resin composition of an isobutylene block copolymer (A) and a thermoplastic polyurethane resin (B) at a weight ratio of (A)/(B) = 1/99 to 99/1.

6. The tube device for insertion into a lacrimal passage according to any one of claims 1 to 5, wherein the opening has a diameter of 0.5 to 0.8 mm.

7. The tube device for insertion into a lacrimal passage according to any one of claims 1 to 6, wherein the predetermined distance is 2 mm or less.
